# EUROPEAN PATENT SPECIFICATION

(11) **EP 4 442 231 B1**
(45) Date of publication and mention of the grant of the patent: **30.09.2026**
(21) Application number: 22900954.3
(22) Date of filing: 21.10.2022
(51) Int. Cl.: A61F 13/49, A61F 13/496

(54) **PANTS-TYPE DIAPER**
HOSENÄHNLICHE WINDEL
COUCHE DE TYPE CULOTTE

(30) Priority: 30.11.2021 JP 2021193831
(43) Date of publication of application: 09.10.2024
(73) Proprietor: DAIO PAPER CORPORATION, Shikokuchuo-shi Ehime 799-0492 (JP)
(72) Inventor: YAMASHITA, Yuichi, Shikokuchuo-shi, Ehime 799-0113 (JP); TAKAGI, Yurika, Shikokuchuo-shi, Ehime 799-0113 (JP)
(74) Representative: Epping - Hermann - Fischer
(86) International application number: PCT/JP2022/039334
(87) International publication number: WO 2023/100521

(56) References cited:
- WO-A1-2017/131024
- WO-A1-2017/159262
- JP-A- 2006 130 042
- JP-A- 2014 133 181
- JP-A- 2014 198 179
- JP-A- 2014 198 180
- JP-A- 2014 207 973
- JP-A- 2015 171 501
- JP-A- 2019 187 959
- JP-A- H03 195 558

## Description

### TECHNICAL FIELD

The present invention relates to a pants-type diaper.

### BACKGROUND ART

As a pants-type diaper, a diaper including an inner member facing the crotch of a wearer, and an outer member bonded to the outer surfaces of a front portion and a back portion of the inner member is known. The above-described outer member is typically composed of a stretchable material in which elastic members are sandwiched between two sheets in a state in which the elastic members are stretched as described in Patent Document 1, for example. After manufacturing, the elastic members contract from the stretched state at the time of manufacturing. At this time, the two sheets on both sides of the elastic members also shrink, and as a result, a plurality of pleats are formed on the two sheets. The elastic members are provided following the circumferential direction of the diaper, and thus the plurality of pleats extend along the up-down direction of the diaper (the up-down direction in a state in which the wearer is standing while wearing the diaper).

### RELATED-ART DOCUMENTS

### PATENT DOCUMENTS

Patent Document 1: Japanese Laid-open Patent Publication No. 2015-211777
Document JP 2014-198180 A describes an elastic structure having straightly extended ridges, wherein a first sheet material and a second sheet material are joined together to form sheet joined sections by an adhesive applied intermittently in an extending direction and continuously in a direction intersecting with the extending direction. According to this document, resilient and elastic members are fixed by the adhesive to at least one of the first sheet material and the second sheet material at intersections with the sheet joined sections, and when the first sheet material and the second sheet material are contracted with contraction of the resilient and elastic members, portions of the first sheet material and the second sheet material positioned between the sheet joined sections are swelled in mutually opposite directions to form the ridges.

### SUMMARY OF THE INVENTION

### PROBLEM TO BE SOLVED BY THE INVENTION

A plurality of pleats formed by sheets extending along the up-down direction as described above enhance the shock absorbing performance (cushioning properties) in the thickness direction of an outer member, and also improve the air permeability. However, for example, if a large amount of excrement is discharged and the excrement discharged to an inner member reaches the outer member, the excrement is likely to move in the up-down direction along the pleats, and may leak from a waist opening that is the upper end of a diaper.

In view of the above, it is an object of one aspect of the present invention to provide a pants-type diaper including a stretchable outer member having a plurality of pleats, in which leakage of excrement from a waist opening can be prevented even when a large amount of excrement or the like is discharged.
The invention is defined by the features of the independent claim. Preferred embodiments are defined in the dependent claims. In the following, parts of the description and drawing referring to aspects or examples, which are not covered by the claims are not presented as embodiments of the invention, but as illustrative examples useful for understanding the invention. The embodiments of the invention are provided by the appended set of claims.

### MEANS TO SOLVE THE PROBLEM

One embodiment of the present disclosure provides a pants-type diaper including: an inner member configured to face a crotch of a wearer; and an outer member configured to be bonded to outer surfaces of a front portion and of a back portion of the inner member and disposed around a lower torso of the wearer, wherein the outer member includes a waist edge portion and a lower waist portion, the waist edge portion having a waist opening at an upper end thereof and the lower waist portion extending downward from a lower end of the waist edge portion, and two sheets and a plurality of elongated elastic members, the plurality of elastic members being arranged in a stretched state between the two sheets and being spaced apart from each other in an up-down direction, wherein, in a state in which the stretched state is released, pleats having a plurality of tips are formed on an inner side and an outer side of the outer member so as to extend in the up-down direction, wherein a space corresponding to a valley between adjacent tips of the plurality of tips does not extend continuously from an uppermost elastic member to a lowermost elastic member of elastic members provided in the waist edge portion among the plurality of elastic members, and wherein a space corresponding to a valley between adjacent tips of the plurality of tips extends continuously from an uppermost elastic member to a lowermost elastic member of elastic members provided in the lower waist portion among the plurality of elastic members.

### EFFECTS OF THE INVENTION

According to one aspect of the present invention, in a pants-type diaper including a stretchable outer member having a plurality of pleats, leakage of excrement from a waist opening can be prevented even when a large amount of excrement is discharged.

### BRIEF DESCRIPTION OF THE DRAWINGS

[FIG. 1] FIG. 1 is a drawing illustrating a pants-type diaper in an unfolded state as viewed from the inner side (skin side);
[FIG. 2] FIG. 2 is drawing illustrating the pants-type diaper in an unfolded state as viewed from the outer side (non-skin side);
[FIG. 3] FIG. 3 is a schematic cross-sectional view of the pants-type diaper taken through line I-I of FIG. 1;
[FIG. 4] FIG. 4 is a schematic cross-sectional view of the pants-type diaper taken through line II-II of FIG. 1;
[FIG. 5] FIG. 5 is a perspective view of the pants-type diaper;
[FIG. 6] FIG. 6 is partially enlarged view of the exterior of an outer member;
[FIG. 7] FIG. 7 is a diagram illustrating the configuration of the outer member;
[FIG. 8] FIG. 8 is a partially enlarged view of a waist edge portion, in which a portion of the waist edge portion is cut away;
[FIG. 9] FIG. 9 is a partially enlarged view of a lower waist portion, in which a portion of the lower waist portion is cut away; and
[FIG. 10] FIG. 10 is an enlarged cross-sectional view of the pants-type diaper taken through line III-III of FIG. 1.

### MODE FOR CARRYING OUT THE DISCLOSURE

Examples of the present disclosure will be described in detail below with reference to the drawings. In the drawings, unless otherwise specified, the same or corresponding components are denoted by the same reference numerals, and the description thereof may be omitted.

### (Basic Structure of Pants-Type Diaper)

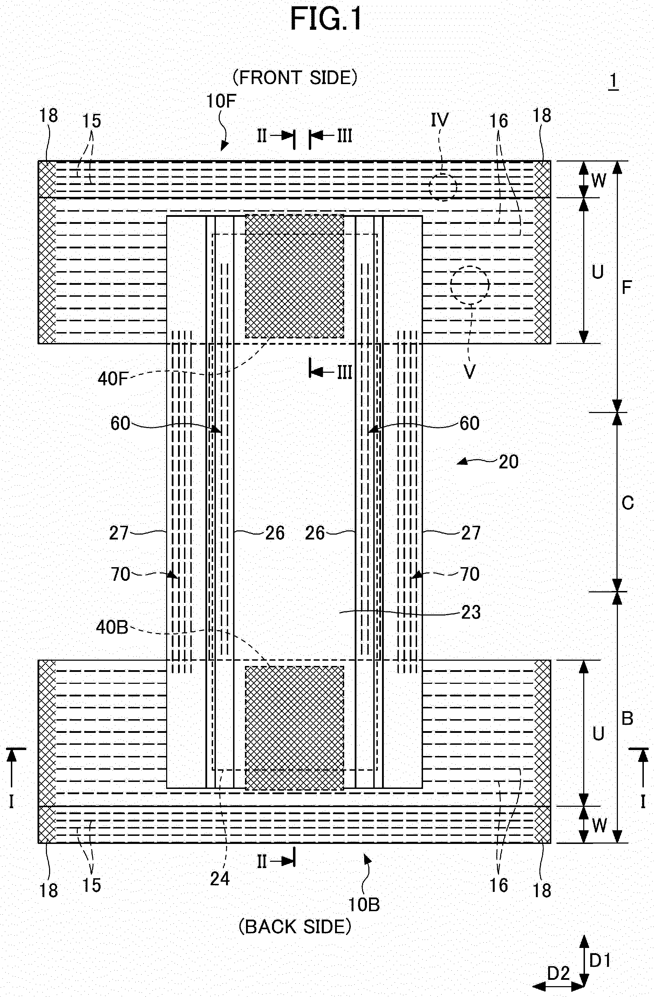

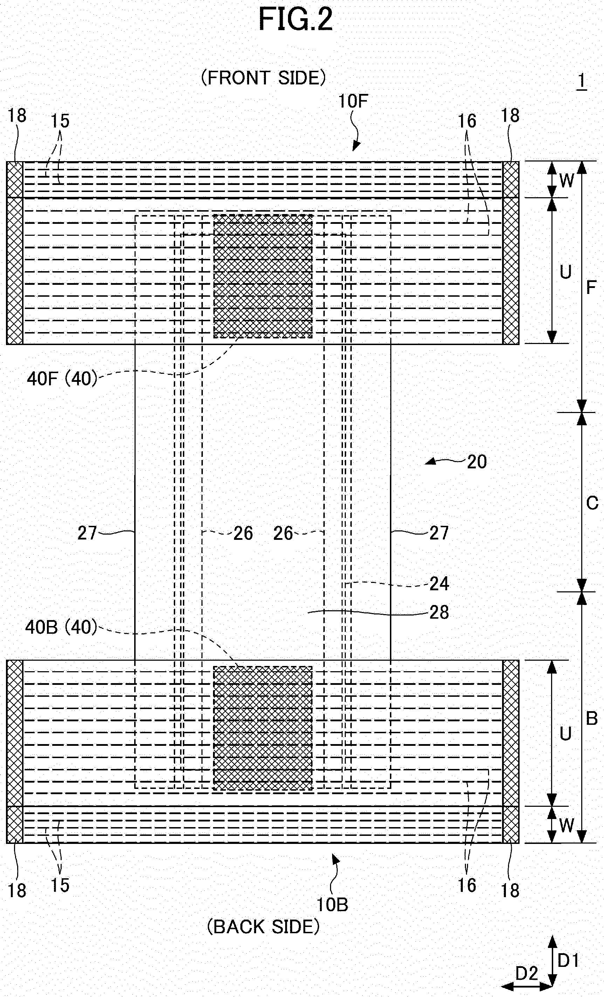

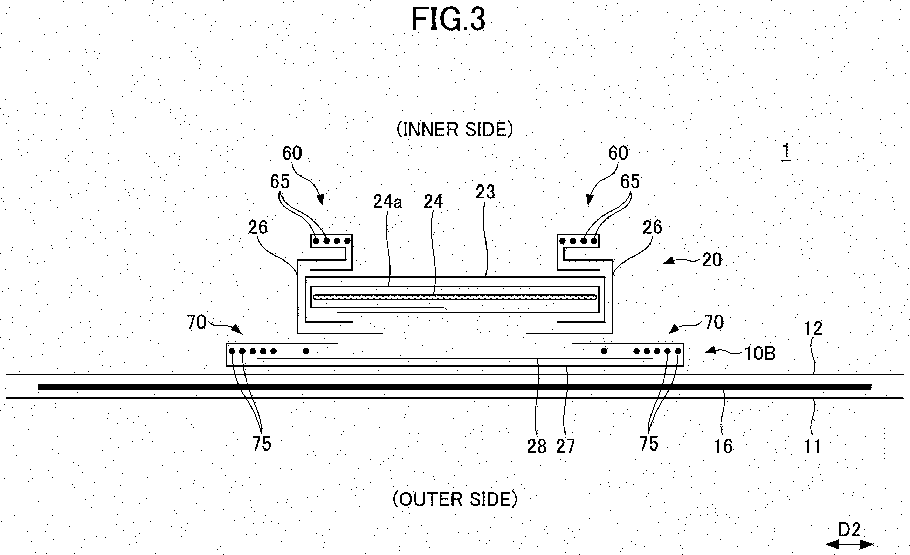

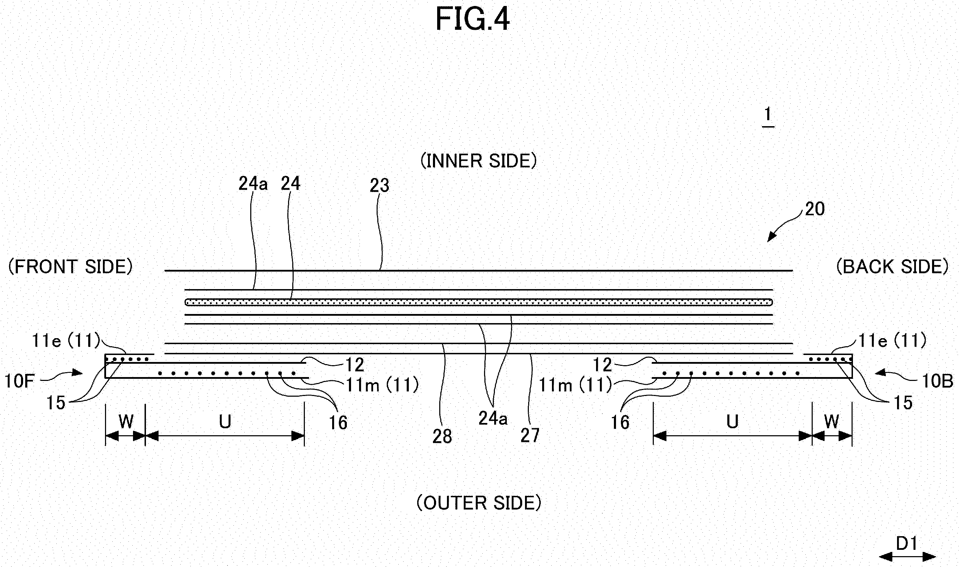

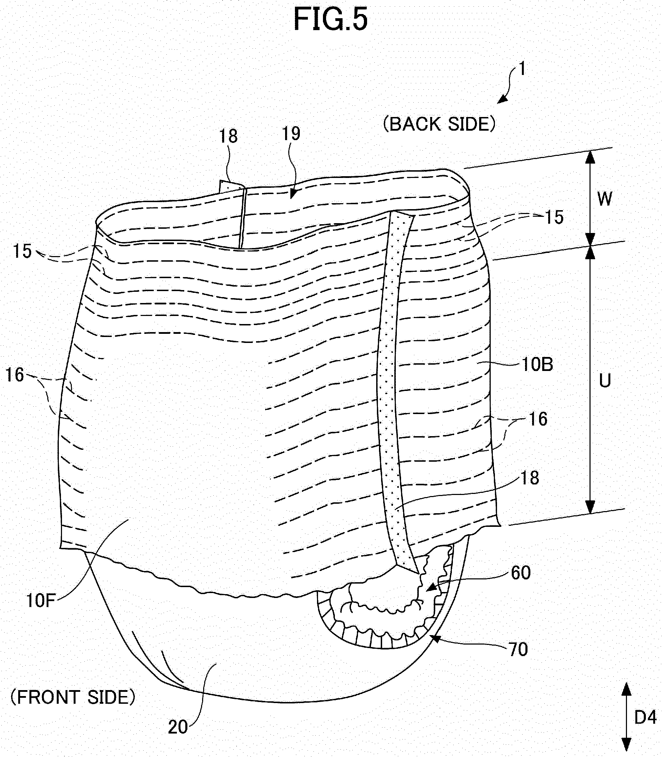

FIG. 1 to FIG. 5 illustrate a pants-type diaper 1. In the present example, a pants-type disposable diaper for a baby is illustrated as an example. FIG. 1 is a drawing illustrating the pants-type diaper 1 in an unfolded state as viewed from the inner surface. FIG. 2 is drawing illustrating the pants-type diaper 1 in an unfolded state as viewed from the outer surface. FIG. 3 is a schematic cross-sectional view of the pants-type diaper taken through line I-I of FIG. 1. FIG. 4 is a schematic cross-sectional view of the pants-type diaper taken through line II-II of FIG. 1. Further, FIG. 5 is a perspective view of the pants-type diaper. As is apparent from the drawings, the pants-type diaper 1 is formed by laminating a plurality of sheet-shaped or layer-shaped members. Further, members of the pants-type diaper 1 may be bonded to each other at a predetermined position or in a predetermined region by an adhesive, fusion, or mechanical bonding means. In the cross-sectional views of FIG. 3 and FIG. 4, the description of a laminated structure of members is given priority, a bonding structure between members is not illustrated, and bonded portions, the arrangement of adhesives, and the like are also not illustrated. A sheet-shaped or layered-shaped member used may be formed of one sheet or may be formed of a laminated sheet obtained by bonding a plurality of sheets.

In the present specification, a side facing the skin when the pants-type diaper 1 is worn is referred to as an inner side or a skin side, and a side exposed to the outside is referred to as an outer side or a non-skin side. Further, a side facing the front side (ventral side) of the body of a wearer when the pants-type diaper 1 is worn is referred to as a front side (ventral side), and a side facing the back side (dorsal side) of the body of the wearer is referred to as a back side (dorsal side). Further, in the present specification, a front-back direction D1 is a direction extending from the front side to the back side (or from the back side to the front side) in a state in which the diaper 1 is unfolded, and a width direction D2 is a direction orthogonal to the front-back direction D1 in a state in which the diaper 1 is unfolded. An up-down direction D4 (FIG. 5) is a direction connecting a waist opening 19 and a crotch portion when the pants-type diaper 1 is worn.

As illustrated in FIG. 1 to FIG. 5, the pants-type diaper 1 is mainly composed of an inner member 20 and an outer member 10 bonded to the outer surface of the inner member 20. The inner member 20 includes an absorber, and includes a portion that faces the crotch of the wearer when the diaper is worn, and absorbs and holds excrement such as urine and feces. The outer member 10 is a portion disposed around the lower torso of the wearer, and holds a front portion and a back portion of the inner member 20 with respect to the body. In the present example, the outer member 10 is not one member, and is divided into two, a front side (ventral side) and a back side (dorsal side). That is, the outer member 10 includes a front outer member 10F and a back outer member 10B. The front outer member 10F and the back outer member 10B are bonded to the outer surfaces of the front portion and the back portion of the inner member 20, respectively.

In the present example, a region corresponding to the crotch of the wearer is referred to as a crotch corresponding region C, a region in front of the crotch corresponding region C is referred to as a front region F, and a region behind the crotch corresponding region C is referred to as a back region B (FIG. 1 and FIG. 2). The front outer member 10F is included in the front region F, and the back outer member 10B is included in the back region B.

Side seal portions 18, 18 are formed by bonding both ends of the front outer member 10F and both ends of the back outer member 10B in the width direction D2 such that the outer member 10 has a tubular shape (FIG. 5). The waist opening 19 through which the lower torso of the wearer passes is defined by the front edge of the front outer member 10F and the rear edge of the back outer member 10B. Further, portions surrounded by the rear edge (lower edge) of the front outer member 10F, the front edge (lower edge) of the back outer member 10B, and the side edges of the inner member 20 serve as foot openings.

### (Inner Member)

In the example illustrated in FIG. 1 and FIG. 2, the inner member 20 has a rectangular shape extending in the front-back direction D1; however, the shape of the inner member 20 is not limited to the illustrated shape. The inner member 20 includes an absorber 24 having an absorption function. As illustrated in FIG. 3 and FIG. 4, a top sheet 23 is provided on the inner side (skin side) of the absorber 24, and a liquid impermeable sheet 28 is provided on the outer side (non-skin side) of the absorber 24. Three-dimensional inner gathers 60 that rise toward the skin are formed on both side portions (both end portions in the width direction D2) of the inner member 20. Further, three-dimensional outer gathers 70 that also rise toward the skin are formed. The three-dimensional outer gathers 70 are located outward of the three-dimensional inner gathers 60 in the width direction D2.

The top sheet 23 is a liquid permeable sheet, and may be, for example, a perforated or non-perforated nonwoven fabric, a porous plastic sheet, or the like. If the top sheet 23 is a nonwoven fabric, examples of raw material fibers used for the nonwoven fabric include synthetic fibers such as olefin-based synthetic fibers, such as polyethylene and polypropylene, polyester-based synthetic fibers, and polyamide-based synthetic fibers; regenerated fibers such as rayon and cuprammonium rayon; and natural fibers such as cotton. Further, a mixed fiber, a composite fiber, or the like using two or more of the above fibers may be used. Further, a processing method of the nonwoven fabric is not particularly limited. Examples of the processing method include a spun lace method, a spun bond method, a thermal bond method, a melt blown method, a needle-punch method, an air-through method, a point bond method, and the like. For example, if flexibility and drapeability are required, the spun bond method or the spun lace method is preferable. If bulkiness and softness are required, the air-through method, the point bond method, or the thermal bond method is preferable.

In order to rapidly transfer liquid that has permeated the top sheet 23 to the absorber 24, an intermediate sheet or a second sheet having a higher liquid permeation rate than that of the top sheet 23 may be provided. The intermediate sheet functions to prevent absorbed liquid from returning from the absorber 24, and also prevent liquid from remaining in the top sheet 23.

The liquid impermeable sheet 28 may be a plastic film made of an olefin-based resin such as polyethylene or polypropylene, a laminated nonwoven fabric in which a plastic film is provide on the surface of a nonwoven fabric, a laminated sheet in which a nonwoven fabric or the like is laminated and bonded onto a plastic film, or the like. Furter, from the viewpoint of preventing stuffiness, a material having liquid impermeability and moisture permeability is favorably used for a back sheet. A plastic film having moisture permeability may be a microporous plastic film obtained by forming a sheet by kneading an inorganic filler in an olefin-based resin such as polyethylene or polypropylene, and subsequently stretching the sheet in a monoaxial or biaxial direction, or the like.

In order to enhance leakage resistance, the liquid impermeable sheet 28 preferably has a size and a shape such that the absorber 24 does not extend beyond the liquid impermeable sheet 28 in a top view.

The absorber 24 can be formed by an assembly of fibers. Such a fiber assembly may be an assembly obtained by accumulating short fibers such as fluff pulp or synthetic fibers, a filament assembly obtained by opening fiber bundles of synthetic fibers such as cellulose acetate as necessary, or the like. It is preferable to disperse and hold super absorbent polymer particles in the absorber 24.

The absorber 24 may have a rectangular shape, or may have a shape whose width (length in the width direction D2) is not constant and varies. For example, the absorber 24 may have a narrower portion at substantially the center in the front-back direction D1 such that the width of the narrower portion is narrower than that of the front and back portions. The dimensions of the absorber 24 can be appropriately determined; however, the absorber 24 preferably extends to a peripheral portion of the inner member 20 or the vicinity thereof in each of the front-back direction D1 and the width direction D2.

The absorber 24 may be surrounded by an encapsulating sheet 24a. The encapsulating sheet 24a may be tissue paper, particularly crepe paper, a nonwoven fabric, a poly-laminate nonwoven fabric, a sheet with small openings, or the like. It is desirable that the encapsulating sheet 24a is a sheet through which super absorbent polymer particles do not pass. Further, the encapsulating sheet 24a is preferably wrapped around the absorber 24 so as to surround the inner surface and the outer surface of the absorber 24, and front and back edge portions of the encapsulating sheet 24a protruding from the front and back sides of the absorber 24 are preferably bonded in the thickness direction of the inner member 20.

Strip-shaped inner gather sheets 26, 26 extending along the front-back direction D1 may be provided on respective side portions of the inner member 20. Each of the inner gather sheets 26, 26 is folded back in the width direction D2, and an elastic member 65 is sandwiched and fixed between sheets of each of the inner gather sheets 26, 26 in a state in which the elastic member 65 is stretched along the front-back direction D1. The elastic member 65 can be fixed by an adhesive, fusion such as heat-sealing of the sheets, or the like.

As the inner gather sheets 26, a nonwoven fabric such as a spun bond nonwoven fabric (SS, SSS, or the like), an SMS nonwoven fabric (SMS, SSMMS, or the like), or a meltblown nonwoven fabric, which is flexible and excellent in uniformity and concealing property and on which water repellent treatment is performed by silicone as necessary, can be suitably used. As the elastic member 65, an elongated elastic member such as a rubber thread can be used.

An outer gather sheet 27 is further disposed on the outer side of the inner member 20. In the present example, the outer gather sheet 27 is disposed outward of the liquid impermeable sheet 28, and is folded back inward so as to wrap around both ends of the liquid impermeable sheet 28 in the width direction D2. At each position where the outer gather sheet 27 is folded back, a plurality of elastic members 75 are sandwiched and fixed between sheets in a state in which the plurality of elastic members 75 are stretched along the front-back direction D1. The outer gather sheet 27 is not necessarily one sheet as illustrated in the drawings, and two outer gather sheets may be provided on respective side portions of the inner member 20. In this case, an additional back sheet may be provided on the outer side of the outer gather sheets. With such a configuration of the outer gather sheet 27, the three-dimensional outer gathers 70 that rise toward the skin are formed at positions outward of the three-dimensional inner gathers 60 in the width direction D2. The three-dimensional inner gathers 60 and the three-dimensional outer gathers 70 can prevent leakage of excrement from around the legs.

### (Outer Member)

As described above, the outer member 10 according to the present example separately includes the front outer member 10F configured to face the front side (ventral side) of the wearer and the back outer member 10B configured to face the back side (dorsal side) of the wearer when the diaper is worn. As illustrated in FIG. 1 and FIG. 2, the front outer member 10F and the back outer member 10B are disposed apart from each other. Therefore, it is not necessary to provide the outer member 10 with a portion facing the crotch, and the front outer member 10F and the back outer member 10B can have a shape such that the front outer member 10F and the back outer member 10B are wrapped around the lower torso of the wearer. Therefore, the front outer member 10F and the back outer member 10B can be formed in a simple shape, for example, a rectangular shape with the longer side being in the width direction D2 as illustrated in FIG. 1 and FIG. 2. However, the shape of the front outer member 10F and the back outer member 10B in a plan view is not limited to a rectangular shape, as long as the front outer member 10F and the back outer member 10B can be wrapped around the lower torso of the wearer. The length of each of the front outer member 10F and the back outer member 10 in the front-back direction D1 (the up-down direction D4 when the diaper is worn) may be 100 mm to 180 mm, and the length of each of the front outer member 10F and the back outer member 10 in the width direction D2 may be 300 mm to 500 mm.

Further, each of the front outer member 10F and the back outer member 10B may include a waist edge portion W and a lower waist portion U. The waist edge portion W constitutes an edge portion having the waist opening 19, and the lower waist portion U is located below the waist edge portion W. The length of the waist edge portion W in the front-back direction D1 (the up-down direction D4 when the diaper is worn) may be 15 mm to 50 mm, and the length of the lower waist portion U in the front-back direction D1 (the up-down direction D4 when the diaper is worn) may be 65 mm to 130 mm.

In the present example, the front outer member 10F and the back outer member 10B have the same size and the same shape, and are disposed symmetrically in the front-back direction. Being that the front outer member 10F and the back outer member 10B have the same configuration, manufacturing time and cost can be reduced. However, the size, the shape, and the arrangement of the front outer member 10F and the back outer member 10B may vary according to the specific application and intended use of the diaper.

The outer member 10 (each of the front outer member 10F and the back outer member 10B) has a stretchable structure that is stretchable in the width direction D2. As illustrated in FIG. 1 to FIG. 4, the stretchable structure is obtained by laminating a first sheet 11 and a second sheet 12 such that the first sheet 11 is located outward of the second sheet 12, and fixing a plurality of elastic members 15, 15,... and 16, 16,... between the first sheet 11 and the second sheet 12 while stretching the plurality of elastic members 15, 15,... and 16, 16,... at a predetermined stretch rate. As used herein, the "stretch rate" refers to a length when the length of an elastic member in a state in which no external force is applied to the elastic member (in a non-pulled state or in a natural state) is set to 100%.

The elastic members 15 and 16 are elongated elastic members such as rubber threads. Further, the elastic members 15 and 16 are spaced apart from each other in the front-back direction D1 (the up-down direction D4 when the diaper is worn), and are arranged such that the stretching direction of the elastic members 15 and 16 is along the width direction D2 (the circumferential direction when the diaper is worn). Note that the elastic members 15, 15,... provided in the waist edge portion W are referred to as edge elastic members, and the elastic members 16, 16,... provided in the lower waist portion U are referred to as lower elastic members.

The number of edge elastic members 15, 15,... may be three to ten. Further, the number of lower elastic members 16, 16,... may be eight to twenty-six.

The material constituting each of the first sheet 11 and the second sheet 12 is not particularly limited. However, the first sheet 11 and the second sheet 12 are both preferably a nonwoven fabric. Examples of raw material fibers of the nonwoven fabric include synthetic fibers such as olefin-based synthetic fibers, such as polyethylene and polypropylene, polyester-based synthetic fibers, and polyamide-based synthetic fibers; regenerated fibers such as rayon and cuprammonium rayon; and natural fibers such as cotton. A mixed fiber, a composite fiber, or the like using two or more of the above fibers may be used. Further, the nonwoven fabric is preferably manufactured by a publicly-known method such as a spun lace method, a spun bond method, a thermal bond method, a melt blown method, a needle-punch method, an air-through method, a point bond method, or the like. A nonwoven fabric manufactured by the spun bond method is preferable because pleats can be easily formed and also the nonwoven fabric can have excellent flexibility.

If the first sheet 11 and the second sheet 12 are a nonwoven fabric manufactured by the spun bond method, a nonwoven fabric on which embossed portions (compressed portions) are formed in advance may be used. Embossed portions are preferably formed such that the flexibility and stretchability of the nonwoven fabric itself are not impaired. For example, embossed portions are preferably formed as not to be continuous in the stretching direction of the elastic members (the edge elastic members 15 and the lower elastic members 16).

Each of the first sheet 11 and the second sheet 12 preferably has a thickness of 0.1 mm to 1 mm and a basis weight of 10 g/m² to 20 g/m². The material, the thickness, and the basis weight of the first sheet 11 may be the same as or different from those of the second sheet 12. In the present specification, the "thicknesses" of a thick member such as an absorber can be measured by using a thickness measuring device (Peacock, Dial Thickness Gauge, Model G-30) manufactured by OZAKI MGF. Co. Ltd., by horizontally placing the sample and the thickness measuring device. The "thickness" of any other thin material such as a nonwoven fabric can be automatically measured by using an automatic thickness measuring device (KES-G5 handy compression measuring program). The thickness of a sheet having holes such as a perforated nonwoven fabric is measured at a portion other than the holes and the edges. Further, in the present specification, the "basis weight" is measured as follows. After a sample or a specimen is dried in advance, the sample or the specimen is left in a test room or a device under normal conditions (a temperature of 23 ± 1°C and a relative humidity of 50 ± 2%), and is put in a constant weight state. A sample is cut off from the specimen in the constant weight state by using a sampling template, and after the weight of the sample is measured, a weight per square meter is calculated.

The elastic members 15 and 16 may be thread-like or string-like elongated elastic members. The material of the elastic members 15 and 16 may be synthetic rubber, natural rubber, a polymer, or the like, or may be a combination of two or more of these. The cross-sectional shape of each of the elastic members is not limited, and may be a circular shape, an elliptical shape, or a quadrangular shape such as a square shape.

As described above, the elastic members 15 and 16 are fixed in a stretched state between the two sheets. In the present specification, fixing the elastic members may mean that the elastic members 15 and 16 are bonded to or adhere to at least one of the first sheet 11 or the second sheet 12. Alternatively, fixing the elastic members 15 and 16 may mean that the elastic members 15 and 16 are not bonded to either the first sheet 11 or the second sheet 12, but the first sheet 11 and the second sheet 12 are bonded to each other. For example, fixing the elastic members 15 and 16 may mean that the elastic members 15 and 16 are fixed by being sandwiched between the first sheet 11 and the second sheet 12. Therefore, fixing the elastic members 15 and 16 can refer to fixing the positions of the elastic members 15 and 16 in the outer member 10, and does not cause the stretchability of the fixed elastic members 15 and 16 to be lost, or does not cause the stretchability of the outer member of the pants-type diaper to be lost even if the stretchability of the elastic members 15 and 16 is partially lost or reduced. In the stretchable structure of the outer member 10 according to the present example, portions where the elastic members and at least one of the first sheet 11 or the second sheet 12 are bonded or portions where the first sheet 11 and the second sheet 12 are bonded are formed, that is, bonded portions are formed. Portions other than the bonded portions are called non-bonded portions.

An adhesive such as a hot melt adhesive may be used to form the bonded portions (see Japanese Laid-open Patent Publication No. 2014-207973, for example). The adhesive may be applied by solid coating, bead coating, curtain coating, summit coating, or spiral coating, or pattern coating (transfer of the adhesive in a letterpress method). Alternatively, the adhesive may be applied to the outer peripheral surfaces of the elastic members by using a comb gun or SureWrap application. Further, fusion such as heat sealing or ultrasonic sealing may be used (Japanese Laid-open Patent Publication No. 2008-154998). The arrangement of the bonded portions is determined according to the desired shapes, sizes, pitches, and the like of pleats.

As described above, the outer member 10 (each of the front outer member 10F and the back outer member 10B) has a structure in which a plurality of elastic members are fixed in a stretched state between the two sheets. In a pre-use product after being manufactured, the stretched state of the plurality of elastic members is released. Upon the release of the stretched state of the elastic members, the elastic members contract in the stretching direction (the longitudinal direction of the elastic members). At this time, portions where the two sheets (the first sheet 11 and the second sheet 12) are not bonded (non-bonded portions) also contract in the stretching direction of the elastic members. The sheets themselves do not have stretchability like the elastic members, the sheets protrude in a direction away from the elastic members in the thickness direction. By such protrusions of the sheets, a plurality of pleats are formed on the surface of the outer member 10. With such a configuration of the outer member 10 having the plurality of pleats (described in detail later) impact resistance in the thickness direction can be improved, a pleasant texture can be obtained, and also air permeability can be improved.

The elastic members 15 and 16 may be provided over the entire outer member 10. However, in adhesion regions 40F and 40B (FIG. 1 and FIG. 2) bonded to the inner member 20, stretching may be reduced even if the elastic members are present. Thus, the elastic members 15 and 16 are preferably provided in regions other than the adhesion regions 40F and 40B. In the present example, the waist edge portion W does not overlap the inner member 20 in a plan view, and does not include a region bonded to the inner member 20. Thus, the edge elastic members 15, 15,... are provided on the entire waist edge portion W. Conversely, the lower waist portion U is bonded to the inner member 20 at substantially the center in the width direction D2. Thus, the lower elastic members 16, 16,... are provided over the entire region of the lower waist portion U other than the adhesion regions 40.

Further, in the present example, as illustrated in FIG. 4, the first sheet 11 disposed outward of the second sheet is folded back inward (to the skin side) so as to cover the edge of the second sheet 12 (wrap around the edge of the second sheet 12) at an end on the waist opening 19 side of the first sheet 11 (at the end in the front-back direction D1 of the diaper 1 in an unfolded state). In the present example, a range in which the edge elastic members 15, 15,... are arranged in the front-back direction D1 corresponds to the range of the waist edge portion W. In the illustrated example, the waist edge portion W has a configuration in which a first sheet main portion 11m, the second sheet 12, and a first sheet folded portion 11e are layered in this order from the outer side to the inner side (FIG. 4). In the illustrated example, the first sheet folded portion 11e is configured so as not to overlap the inner member 20. However, unlike the illustrated example, the length of the first sheet folded portion 11e in the front-back direction D1 may be increased, and the first sheet folded portion 11e may overlap the inner member 20 in a plan view. More specifically, the first sheet folded portion 11e may overlap the absorber 24 in a plan view. Further, the first sheet 11 is not necessarily folded back at the waist opening 19, and the edge of the first sheet 11 and the edge of the second sheet 12 may be bonded to each other along the waist opening 19. However, by folding back the sheet, the edge of the sheet is not exposed, and thus the texture of the edge of the sheet near the waist opening 19 is improved, and also fibers included in a nonwoven fabric can be prevented from coming off from the waist opening 19. Further, instead of the first sheet 11, the second sheet 12 may be folded back outward.

### (Pleated Structures of Outer Member)

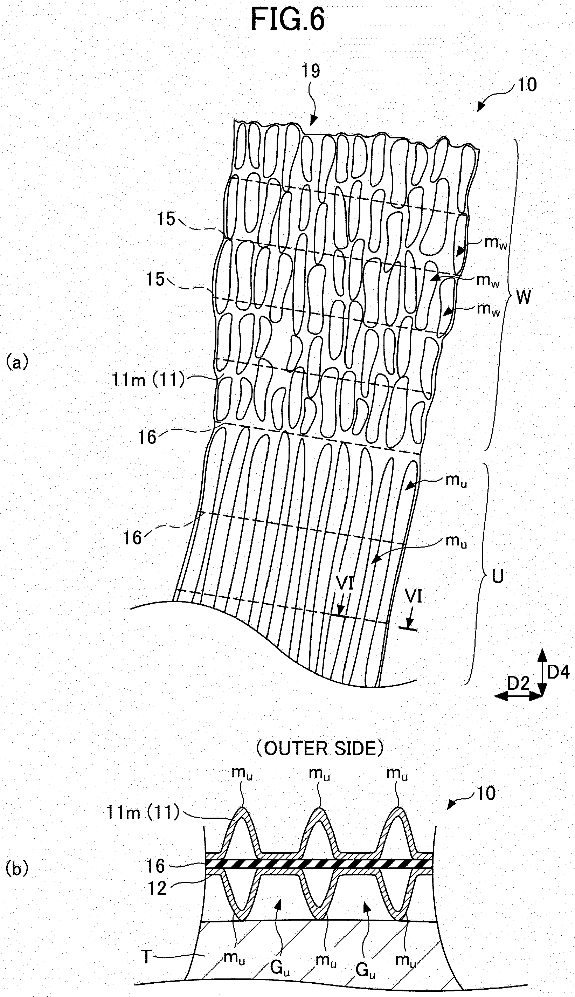

Pleated structures of the outer member according to the present example will be described in more detail. FIG. 6 is a partially enlarged view of the exterior of the outer member 10. FIG. 6 illustrates a state in which the stretched state of the elongated elastic members 15 and 16 extending along the width direction D2 are released, and the sheets (the first sheet 11 and the second sheet 12) sandwiching the elastic members 15 and 16 are gathered in the width direction D2, thereby forming pleats. In both the waist edge portion W and the lower waist portion U, a plurality of pleats extending along the up-down direction D4 (front-back direction D1) are arranged side by side in the width direction D2. However, each of pleat tips m_{W}, m_{W},... formed in the waist edge portion W does not extend across the entire length of the waist edge portion W in the up-down direction D4, and is interrupted at an intermediate position in the up-down direction D4. In contrast, each of pleat tips m_{U}, m_{U},... formed in the lower waist portion U extends across the entire length of the lower waist portion U in the up-down direction D4 so as to extend at least in a range from the uppermost lower elastic member 16 to the lowermost lower elastic member 16 of the lower elastic members 16, 16,....

A plurality of pleats are formed on both the inner surface and the outer surface of the outer member 10. FIG. 6 (b) illustrates a cross section of the lower waist portion U, that is, a schematic view of a cross section of the lower waist portion U taken through line VI-VI of FIG. 6 (a), as viewed from above. As illustrated in FIG. 6 (b), in the lower waist portion U, pleat tips m_{U}, m_{U},... extending along the up-down direction D4 protrude inward, and thus, portions of the outer member 10 contact the skin T of the wearer. A valley is formed between adjacent pleat tips m_{U}, m_{U} and a space GU is present between the valley and the skin T. When each of the pleat tips m_{U}, m_{U},... extends continuously across the entire length of the lower waist portion U in the up-down direction D4, a valley between adjacent pleat tips also extends continuously in the up-down direction D4, and a space GU extends continuously in the up-down direction D4. By causing such spaces GU formed by valleys between the pleat tips m_{U}, m_{U},... to extend continuously in the up-down direction D4 (front-back direction D1), cushioning properties in the thickness direction can be obtained by a plurality of pleats, and also air permeability can be obtained across the entire length of the lower waist portion U in the up-down direction D4.

Typically, excrement from the wearer is received and held by the inner member 20. However, for example, if a large amount of excrement is discharged or rapid excretion occurs, and the excrement reaches the outer member 10, there would be a possibility that the excrement would leak from the waist opening 19. In such a case, if a space formed by a valley between adjacent pleat tips is continuous along the up-down direction D4 and reaches to the vicinity of the waist opening 19, the excrement would move upward through the space. In light of this, according to the present example, the waist edge portion W is configured such that each of the pleat tips m_{W}, m_{W},... does not extend continuously across the entire length of the waist edge portion W in the up-down direction D4, that is, each of the pleat tips m_{W}, m_{W},... is interrupted at an intermediate position in the up-down direction D4 or is bent in another direction (FIG. 6 (a)). Therefore, unlike the configuration of the lower waist portion U, a space (not illustrated) between adjacent pleat tips m_{W}, m_{W} does not extend continuously from the uppermost edge elastic member 15 to the lowermost edge elastic member 15 of the edge elastic members 15, 15,.... Accordingly, even if excrement moves upward from the lower waist portion U to the waist edge portion W, the excrement can be blocked at an intermediate position of the waist edge portion W in the up-down direction D4, and thus leakage of the excrement from the waist opening 19 can be prevented.

As described above, according to the present example, the structure of the waist edge portion W differs from the structure of the lower waist portion U. While the structure of the waist edge portion W can prevent leakage of excrement from the waist opening 19, the structure of the lower waist portion U can ensure cushioning properties and air permeability.

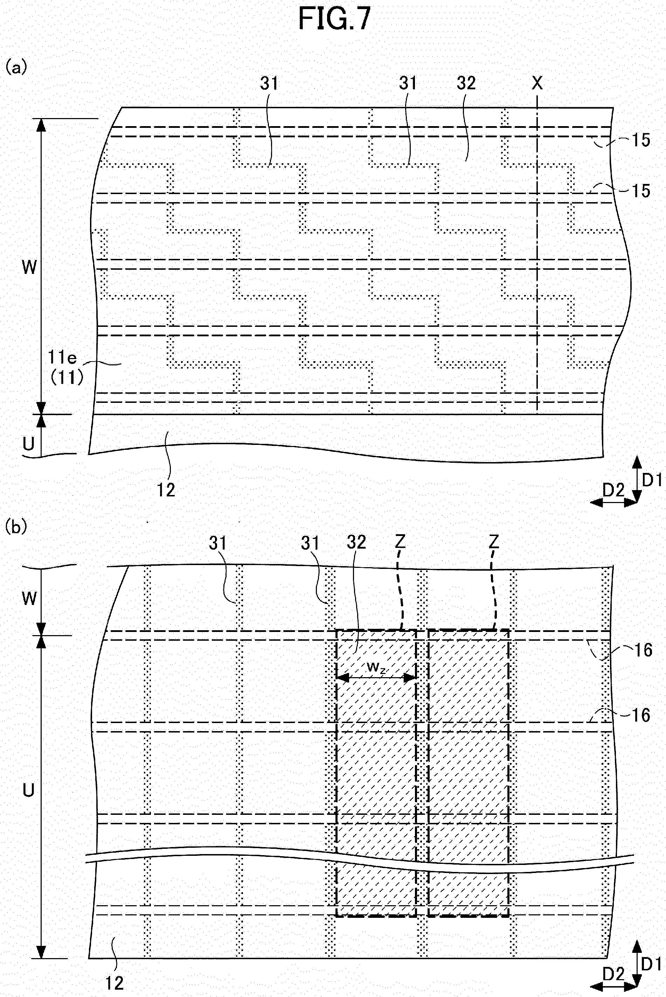

Further, elements that bring about the difference in the above-described structures (pleated structures) between the waist edge portion W and the lower waist portion U will be described. FIG. 7 (a) illustrates a portion IV of FIG. 1, that is, an enlarged view of a portion of the waist edge portion W. FIG. 7 (b) illustrates a portion V of FIG. 1, that is, an enlarged view of a portion of the lower waist portion U. FIG. 7 illustrates the stretched state of elastic members. Note that the following description is based on the enlarged views of the portion IV and the portion V, which are portions of the front outer member 10F. However, in the present example, the front outer member 10F and the back outer member 10B have the same configuration, and thus the following description is applicable to both the front outer member 10F and the back outer member 10B.

As illustrated in FIG. 7 (a), in the waist edge portion W, edge elastic members 15, 15,... are arranged between the first sheet folded portion 11e and the second sheet 12. In the waist edge portion W, bonded portions 31, 31,... are formed by bonding the first sheet folded portion 11e and the second sheet 12, and a region other than the bonded portions 31, 31,... is a non-bonded portion 32 in a plan view. Similarly, as illustrated in FIG. 7 (b), in the lower waist portion U, lower elastic members 16, 16,... are arranged between the first sheet main portion 11m (illustrated in FIG. 3 and FIG. 4) and the second sheet 12. In the lower waist portion U, bonded portions 31, 31,... are formed by bonding the first sheet main portion 11m and the second sheet 12, and a region other than the bonded portions 31, 31,... is a non-bonded portion 32 in a plan view. The bonded portions 31, 31,... illustrated in FIG. 7 (a) and FIG. 7 (b) are bonded portions between the sheets. Further, for convenience of description, in FIG. 7 (a) and FIG. 7 (b), the edge elastic members 15, 15,... and the lower elastic members 16, 16,... have the same thickness, and also the edge elastic members 15, 15,... and the lower elastic members 16, 16,... have the same pitch in the front-back direction D1 (in the up-down direction D4 when the diaper is worn).

In the waist edge portion W illustrated in FIG. 7 (a), each of the bonded portions 31, 31,... is formed in a line shape extending in the front-back direction D1 as a whole. Specifically, in the waist edge portion W, each of the bonded portions 31, 31,... is formed in a stepped shape. In the lower waist portion U illustrated in FIG. 7 (b), each of the bonded portions 31, 31,... is formed in a line shape as a whole. In the lower waist portion U, the bonded portions 31, 31... are spaced apart from each other in the width direction D2, and extend along the front-back direction D1.

In the lower waist portion U (FIG. 7 (b)), an imaginary strip-shaped region Z extending across the entire length of the lower waist portion U in the front-back direction D1 and having a constant width w_{Z} is assumed to be formed in the non-bonded portion 32, that is, in a region that does not include the bonded portions 31. The strip-shaped region Z is a region formed over all the lower elastic members 16, 16,... included in the lower waist portion U, and can be a region extending at least from the uppermost lower elastic member to the lowermost lower elastic member. The non-bonded portion 32 is a portion where the sheets (the first sheet main portion 11m and the second sheet 12) in the strip-shaped region Z protrude so as to be separated from the lower elastic members 16, 16,... when the lower elastic members 16, 16,... contract, and as a result, a pleat tip (m_{U} in FIG. 6) can be formed. Because the strip-shaped region Z that does not include the bonded portions 31 can be formed over substantially the entire lower waist portion U in the front-back direction D1, a plurality of long and continuous pleats can be formed so as to extend across substantially the entire length of the lower waist portion U in the front-back direction D1 (the lower waist portion U in FIG. 6). Further, a space (G_{U} in FIG. 6) between adjacent pleat tips can be formed continuously in the front-back direction D1, and thus air permeability in the front-back direction D1 can be improved.

The above-described constant width w_{Z} of the imaginary strip-shaped region Z can be 2 mm to 8 mm in a state in which the lower elastic members 16, 16,... are stretched. By setting the constant width in this range, a sheet portion having a sufficient width can be obtained such that the non-bonded portion 32 protrudes between adjacent bonded portions 31, 31, thereby forming a pleat tip. Further, the formed pleat tip is less likely to collapse.

In the example illustrated in FIG. 7 (b), the bonded portions 31, 31,... formed in the lower waist portion U are spaced apart from each other in the width direction D2, and are each formed in a linear shape extending along the front-back direction D1. As used herein, the phrase "along the front-back direction D1" may include a direction that forms an angle of 10° and preferably 5° with respect to the front-back direction D1. Further, each of the bonded portions 31, 31,... may be continuous in the front-back direction D1, or may be a plurality of dots extending along the front-back direction D1.

In the waist edge portion W (FIG. 7 (a)), it is preferable that a strip-shaped region Z having a constant width w_{Z} as formed in the lower waist portion U is not formed in the non-bonded portion 32. It is more preferable that, even in the case of a strip-shaped region having a constant width w_{Z} of less than 2 mm, such a strip-shaped region is not formed in the non-bonded portion 32. That is, a region that does not include a bonded portion 31 and extends across all the edge elastic members 15, 15,... or at least from the uppermost edge elastic member to the lowermost edge elastic member cannot be formed in the waist edge portion W. In the example of FIG. 7 (a), according to the invention, when a given imaginary straight line X is drawn along the front-back direction D1 (the up-down direction D4 when the diaper is worn), the given imaginary straight line X intersects bonded portion(s) 31. Therefore, a pleat tip (m_{W} in FIG. 6) formed in the waist edge portion W is not continuous along the front-back direction D1, and is divided at an intermediate position in the front-back direction D1 or is bent so as to deviate from the front-back direction D1.

A method of forming bonded portions 31 used in each of the configurations of the waist edge portion W illustrated in FIG. 7 (a) and the lower waist portion U illustrated in FIG. 7 (b) is not particularly limited, and either an adhesive or fusion as described above can be used, or both an adhesive and fusion can be used in some cases. Note that as illustrated in FIG. 7 (a) and FIG. 7 (b), bonded portions 31 may be configured not to overlap the elastic members 15 and 16 in a plan view, or bonded portions 31 may be configured to overlap some or all of the elastic members 15 and 16 in a plan view.

In the present example, the edge elastic members 15, 15,... provided in the waist edge portion W may be fixed by an adhesive, and the lower elastic members 16, 16,... provided in the lower waist portion U may be fixed by fusion. Such a configuration in which a method of forming bonded portions differs between the waist edge portion W and the lower waist portion U as described above provides the following effects.

If the lower elastic members 16, 16,... in the lower waist portion U are fixed by fusion, for example, by ultrasonic sealing, the rigidity or hardness of bonded portions 31, 31,... formed by fusion clearly differs from that of adjacent non-bonded portions 32. Thus, the sheets (the first sheet main portion 11m and the second sheet 12) can be easily bent at the boundaries between the bonded portions 31 and the non-bonded portions 32, and pleat tips can be easily formed along the front-back direction D1 when the lower elastic members 16, 16,... contract. Accordingly, by utilizing fusion as a method for forming bonded portions in the lower waist portion U, cushioning properties by pleats and air permeability can be improved.

Further, even when the stretch rate of elastic members provided in the waist edge portion W and the stretch rate of elastic members provided in the lower waist portion U are different, the difference in the stretch rates can be less noticeable when the diaper is worn, by fixing elastic members by an adhesive, particularly by applying an adhesive to the outer peripheral surfaces of the elastic members (with a comb gun, SureWrap application, or the like) in the waist edge portion W, and fixing the elastic members by fusion in the lower waist portion U.

Further, the following effects can be obtained by fixing the edge elastic members 15, 15,... by an adhesive in the waist edge portion W, and fixing the elastic members 16, 16,... by fusion in the lower waist portion U. In the present example, the stretchable structure of the outer member 10 provides a good fit to the lower torso of the wearer. In particular, the stretchable structure provides an improved fit in the vicinity of the opening 19 where close contact is required (having no looseness is required), while allowing the diaper to be easily put on or taken off and to have good wearability. More specifically, in a region where elastic members are fixed by an adhesive, the stretchability of the elastic members tends to be suppressed particularly when the adhesive adheres to the elastic members. Therefore, if elastic members are fixed by an adhesive in the entire outer member 10, a larger force would be required to stretch the elastic members, for example, to widen the waist opening 19 when the diaper is put on or taken off. This can prevent the diaper from being loosened especially at the waist opening and can prevent the diaper from slipping down. Conversely, if some elastic members in the outer member 10 are fixed by fusing the sheets, the stretchability of the elastic members is easily maintained. Therefore, by fixing elastic members by fusion in the lower waist portion U, the outer member can be smoothly stretched even with a small force. Therefore, a pants-type diaper that can be easily put on and taken off can be obtained. Further, by using a combination of an adhesive and fusion to fix elastic members, the amount of the adhesive to be used can be reduced, and the cost can be reduced.

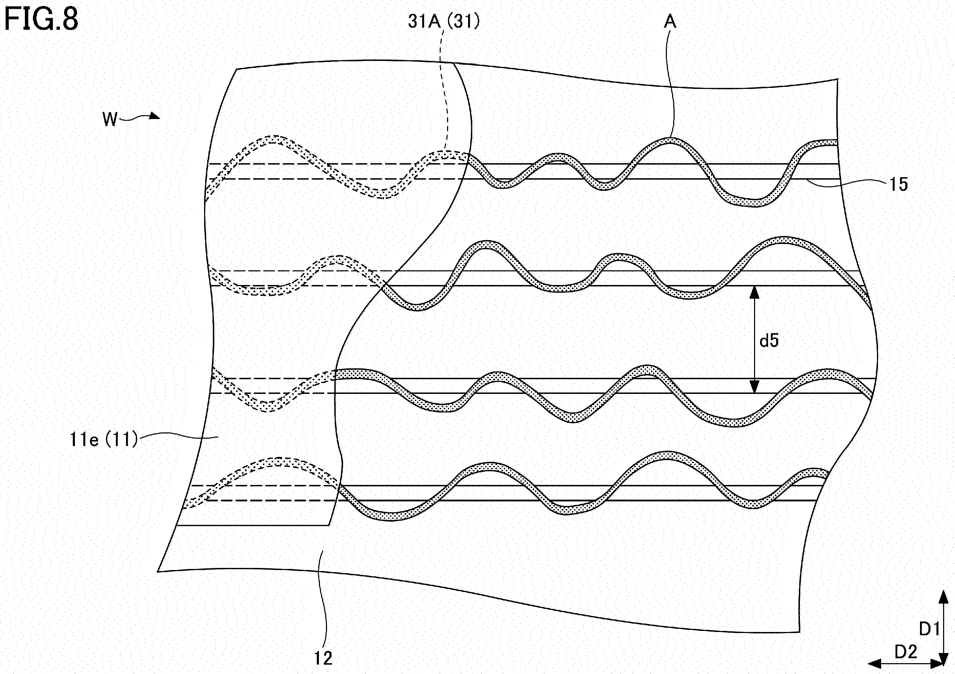

FIG. 8 illustrates an example in which edge elastic members 15, 15,... are fixed by an adhesive A in the waist edge portion W. FIG. 8 is a partial cutaway view. In the example illustrated in FIG. 8, the stretchable direction of the edge elastic members 15, 15,... is along the width direction D2, and the edge elastic members 15, 15,... are spaced apart from each other in the front-back direction D1. The adhesive A is applied so as to partially overlap a corresponding edge elastic member of the edge elastic members 15, 15,.... In the illustrated example, the adhesive A having a line shape in a plan view is applied continuously along the width direction D2. More specifically, the adhesive A has a wave shape having an amplitude in the front-back direction D1 and extending in the width direction D2. In the example illustrated in FIG. 8, in a plan view, a wave-shaped portion where the adhesive A is applied serves as a bonded portion 31A formed by the adhesive, and a portion other than the bonded portion 31A serves as a non-bonded portion 32.

In the waist edge portion W illustrated in FIG. 8, the bonded portion 31A extends along the width direction D2. Thus, as with the case of FIG. 7 (a), an imaginary strip-shaped region Z (FIG. 7 (b)) having a constant width from the uppermost edge elastic member to the lowermost edge elastic member of the edge elastic members 15, 15,... is not formed in the non-bonded portion 32. Further, similarly, if a given imaginary straight line X is drawn along the front-back direction D1 in the waist edge portion W illustrated in FIG. 8, the imaginary straight line X intersects the adhesive A. Therefore, even when the edge elastic members 15, 15,... of the waist edge portion W contract and pleats are formed, each of the pleats is less likely to be continuous in the front-back direction D1, and is likely to be divided by the bonded portion 31A at an intermediate position in the front-back direction D1. Accordingly, even when excrement reaches the outer member 10, leakage of the excrement along the pleats in the front-back direction D1 can be suppressed.

Note that the shape of the adhesive A is not limited to the wave shape as illustrated in FIG. 8, and may be a zigzag shape. Further, the adhesive A is not necessarily continuous in the width direction D2 as illustrated in FIG. 8, and may be discontinuous at an intermediate position in the width direction D2.

Line-shaped adhesives A (bonded portions 31A) adjacent to each other in the width direction D2 may have the same shape or different shapes. More specifically, when each of adhesives A is applied in a wave shape as illustrated in FIG. 8, adjacent adhesives A may have different waveforms. That is, one or more of the amplitude, the wavelength, and the phase of a wave of one of the adjacent adhesives A may be different from the other adhesive A. If the adjacent adhesives A (bonded portions 31A) have different waveforms, bonded portions 31A are formed randomly in the entire waist edge portion W. Pleats formed in the waist edge portion W by the randomly-formed bonded portions 31 become irregular, as compared to pleats extending regularly across the entire length of the lower waist portion U in the front-back direction D1. Accordingly, the effect of preventing movement of excrement along the front-back direction D1 can be improved.

As described above, when the adhesives A are used to fix the elastic members, hot melt adhesives are suitably used as the adhesives. Examples of the hot melt adhesives include EVA-based adhesives, adhesion rubber-based (elastomer-based) adhesives, olefin-based adhesives, and polyester/polyamide-based adhesives. The adhesion rubber-based (elastomer-based) adhesives are preferable.

As illustrated in FIG. 8, the pitch d5 between edge elastic members 15, 15 can be 4 mm to 12 mm, and preferably 4 mm to 8 mm. By increasing the pitch d5, pleats formed in the front-back direction D1 become unstable, and thus the continuity of the pleats in the front-back direction D1 is reduced. Therefore, the above-described effect of preventing excrement from moving along the front-back direction D1 can be improved.

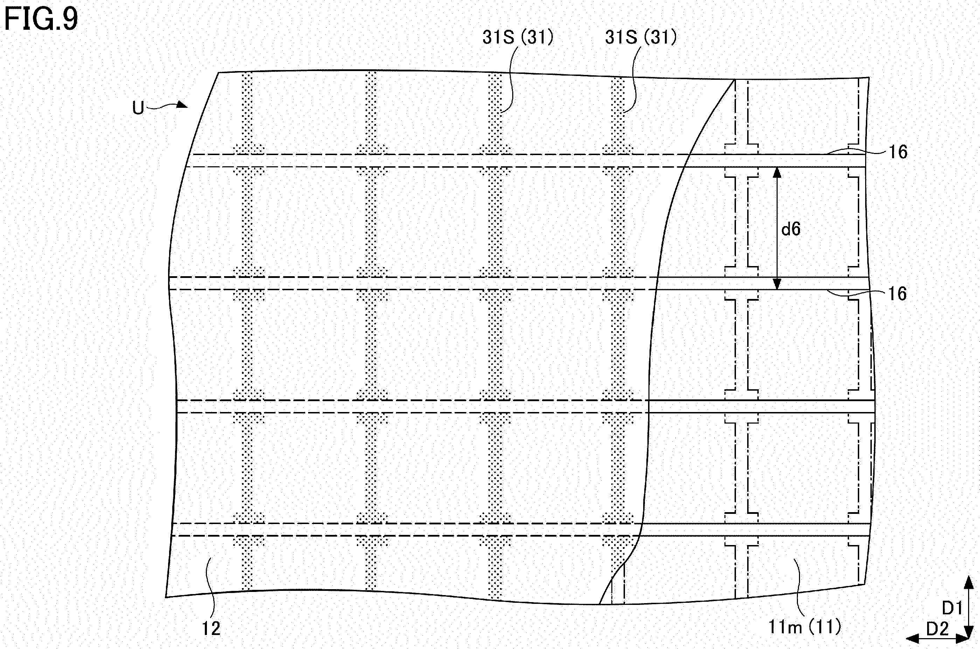

Next, FIG. 9 illustrates an example in which lower elastic members 16, 16,... in the lower waist portion U are fixed by fusion. FIG. 9 illustrates a configuration in which the lower elastic members 16, 16,... are stretched (no pleats are formed on the two sheets). In the example illustrated in FIG. 9, fusion-bonded portions 31S, 31S,... are formed in a linear shape extending along the front-back direction D1, and are spaced apart from each other in the width direction D2. The pitch between adjacent fusion-bonded portions 31S, 31S in the width direction D2 may be 4 mm to 8 mm. Each of the fusion-bonded portions 31S, 31S,... may be formed intermittently at the positions of the lower elastic members 16, 16,... as in the illustrated example, or may be formed so as to overlap some or all of the lower elastic members 16, 16,... in a plan view. The bonded portions 31S, 31S,... are preferably formed so as to suppress breakage of strands due to damage to the lower elastic members 16, 16,....

The pitch d6 between lower elastic members 16, 16 can be 3 mm to 9 mm, and preferably 4 mm to 6 mm. The pitch d6 may be larger than, smaller than, or equal to the pitch d5 between the edge elastic members 15, 15. However, if the pitch d6 between the lower elastic members 16, 16 is smaller than the pitch d5 between the edge elastic members 15, 15, changes in the heights of pleat tips of the lower waist portion U in a direction intersecting the stretching direction of the elastic members can be suppressed.

Further, in the example illustrated in FIG. 9, the widths (lengths in the width direction D2) of the linear bonded portions 31S, 31S,... are not constant, and are increased in the vicinity of the lower elastic members 16, 16,.... This is preferable because the lower elastic members 16, 16,... can be more reliably sandwiched in the front-back direction D1.

The thickness of each of the elastic members 15 and 16 may be 310 dtex to 1,240 dtex, and preferably 470 dtex to 940 dtex. The edge elastic members 15 and the lower elastic members 16 may have the same thickness or different thicknesses.

The elastic members 15 and 16 can be arranged at a stretch rate of 200% to 350%. Further, the edge elastic members 15, 15,... are preferably arrange at a stretch rate of 220% to 260%, and the lower elastic members 16, 16,... are preferably arrange at a stretch rate of 260% to 300%. The stretch rate of the edge elastic members 15, 15,... and the stretch rate of the lower elastic members 16, 16,... may be the same or may be different. Further, the stretch rate of the edge elastic members 15, 15,... is preferably smaller than the stretch rate of the lower elastic members 16, 16,.... Accordingly, the outer member 10 is less likely to slip down along the slope of the abdomen (from the abdomen to the crotch portion) of the wearer. As described above, even when the stretch rate of the elastic members in the waist edge portion W and the stretch rate of the elastic members in the lower waist portion U are different, the difference in the stretch rates can be made less noticeable when the diaper is worn, by fixing the elastic members in the waist edge portion W by an adhesive, particularly by applying an adhesive to the outer peripheral surfaces of the elastic members (with a comb gun, SureWrap application, or the like), and fixing the elastic members in the lower waist portion U by fusion.

Further, the front outer member 10F and the back outer member 10B may have different configurations of elastic members 15 and 16. For example, the thicknesses of elastic members 15 and 16 of the front outer member 10F, particularly the thicknesses of the lower elastic members 16 of the front outer member 10F can be increased as compared to those of the back outer member 10B. As a result, the diaper can more tightly and closely fit a lower region such as the inguinal region on the ventral side of the wearer. Further, all elastic members in the waist edge portion do not necessarily have the same thickness and the same stretch rate. For example, the thickness and the stretch rate of elastic members in the upper part of the waist edge portion W may be different from the thickness and the stretch rate of elastic members in the lower part of the waist edge portion W.

According to one example of the present disclosure, a pants-type diaper includes an inner member configured to face a crotch of a wearer; and an outer member configured to be bonded to outer surfaces of a front portion and a back portion of the inner member and disposed around a lower torso of the wearer. The outer member includes a waist edge portion having a waist opening at an upper end thereof and a lower waist portion extending downward from a lower end of the waist edge portion. The outer member includes two sheets and a plurality of elongated elastic members. The plurality of elastic members are disposed in a stretched state between the two sheets and are spaced apart from each other in an up-down direction. An edge elastic member provided in the waist edge portion, among the plurality of elastic members, is fixed by an adhesive, and a lower elastic member provided in the lower waist portion, among the plurality of elastic members, is fixed by fusion of the two sheets.

According to the above-described example, elastic members 15, 15,... in a waist edge portion W are fixed by an adhesive, particularly by applying an adhesive to the outer peripheral surfaces of the elastic members 15, 15,... (with a comb gun, SureWrap application, or the like), elastic members 16, 16,... in a lower waist portion U are fixed by fusion. Thus, even when the stretch rate of the elastic members in the waist edge portion W and the stretch rate of the elastic members in the lower waist portion U are different, the difference in the stretch rates is less likely to be noticeable when the diaper is worn. Further, the elastic members 16, 16,... provided in the lower waist portion U are fixed by fusing the two sheets instead of using an adhesive, and thus the rigidity of bonded portions formed by fusing the sheets is higher than the rigidity of adjacent non-bonded portions, thereby allowing the sheets to be easily bent at the boundaries between the bonded portions and the non-bonded portions. Therefore, pleat tips can easily rise, and thus pleats extending continuously along the up-down direction can be reliably formed. Accordingly, shock absorbing performance (cushioning properties) in the thickness direction can be obtained in the up-down direction by the pleat tips, and air can easily pass through spaces between the skin and valleys between the pleats tips, thereby improving air permeability.

Further, when the elastic members 15, 15,... in the waist edge portion W are fixed by an adhesive, the adhesive may be applied by any of the above-described various methods such as a method of applying the adhesive to the sheets and a method of applying the adhesive to the outer peripheral surfaces of the elastic members. Therefore, by appropriately selecting an application method, bonded portions can be formed at random in the waist edge portion W. In addition, a configuration in which a space, corresponding to a valley between adjacent ones of a plurality of pleat tips extending in the up-down direction, is not continuous from the uppermost elastic member to the lowermost elastic member of the plurality of elastic members can be easily formed. Accordingly, leakage from the waist opening can be prevented.

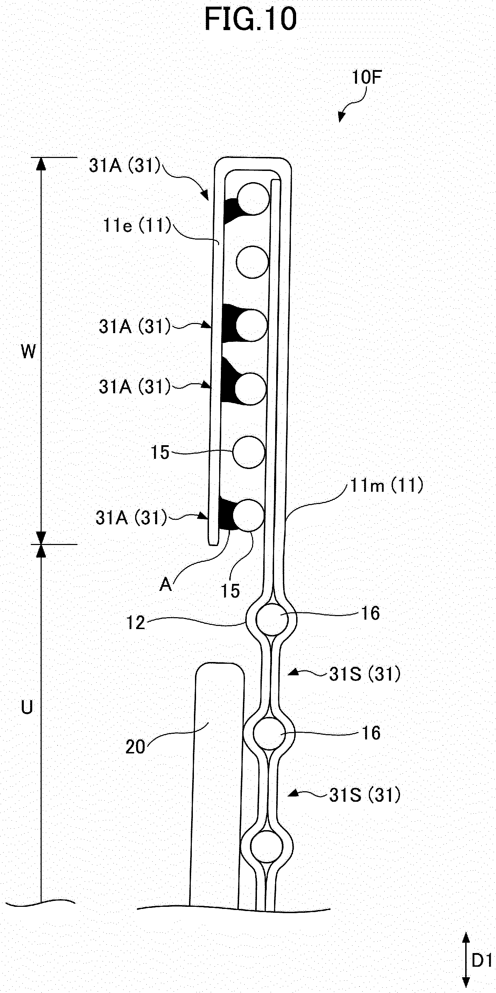

As illustrated in FIG. 1 to FIG. 4, the first sheet 11 is folded back inward so as to wrap around the edge on the waist opening 19 side of the second sheet 12. FIG. 10 is a cross-sectional view of a portion ranging from the waist edge portion W to an upper portion of the lower waist portion U, that is, a cross-sectional view taken through line III-III of FIG. 1. As illustrated in FIG. 10, the first sheet folded portion 11e, the second sheet 12, and the first sheet main portion 11m are layered in this order from the inner side to the outer side of the waist edge portion W. In this manner, in the waist edge portion W, three layers of sheets are laminated, and the edge elastic members 15, 15,... are arranged between the first sheet folded portion 11e and the second sheet 12. Conversely, the lower waist portion U has a structure in which two layers of the first sheet 11 and the second sheet 12 are laminated, and the lower elastic members 16, 16,... are arranged between the sheets.

As described above, in the present example, positions where the edge elastic members 15, 15,... are provided in the waist edge portion W and positions where the lower elastic members 16, 16,... are provided in the lower waist portion U are different in the thickness direction. More specifically, the edge elastic members 15, 15,... are provided inward relative to the lower elastic members 16, 16,.... That is, the edge elastic members 15, 15,... are provided at positions closer to the skin of the wearer than the lower elastic members 16, 16,... are. Therefore, the edge elastic members 15, 15,... can more effectively exert their contraction force. Accordingly, the close contact can be achieved in the vicinity of the waist opening 19, and leakage of excrement from the waist opening 19 can be prevented.

Although the present disclosure has been described above based on specific examples, the present disclosure is not limited to these examples. Further, various changes, modifications, substitutions, additions, deletions, and combinations can be made to the above-described examples within the scope defined by the claims, which are included within the technical scope of the present disclosure.

Further, specific aspects of the present disclosure will be described below.

### (Example 1)

In an aspect according to Example 1, a pants-type diaper includes: an inner member configured to face a crotch of a wearer; and an outer member configured to be bonded to outer surfaces of a front portion and of a back portion of the inner member and disposed around a lower torso of the wearer, wherein the outer member includes a waist edge portion and a lower waist portion, the waist edge portion having a waist opening at an upper end thereof and the lower waist portion extending downward from a lower end of the waist edge portion, and two sheets and a plurality of elongated elastic members, the plurality of elastic members being arranged in a stretched state between the two sheets and being spaced apart from each other in an up-down direction, wherein, in a state in which the stretched state is released, pleats having a plurality of tips are formed on an inner side and an outer side of the outer member so as to extend in the up-down direction, wherein a space corresponding to a valley between adjacent tips of the plurality of tips does not extend continuously from an uppermost elastic member to a lowermost elastic member of elastic members provided in the waist edge portion among the plurality of elastic members, and wherein a space corresponding to a valley between adjacent tips of the plurality of tips extends continuously from an uppermost elastic member to a lowermost elastic member of elastic members provided in the lower waist portion among the plurality of elastic members.

In the aspect according to Example 1, a space corresponding to a valley between tips of adjacent pleats extending in the front-back direction does not extend continuously from an uppermost elastic member to a lowermost elastic member of elastic members provided in the waist edge portion. That is, each pleat formed in the waist edge portion does not extend continuously across the entire length of the waist edge portion in the up-down direction. Therefore, for example, if a large amount of excrement is discharged and the excrement reaches the outer member, the excrement can be prevented from moving through the space between the skin and the valley or the moving speed of the excrement can be reduced on the inner side of the waist edge portion. The waist edge portion having the above-described configuration can reliably block the excrement in the vicinity of the waist opening, and can prevent the excrement from leaking to the outside of the diaper. Conversely, a valley between tips of adjacent pleats of extends continuously from an uppermost elastic member to a lowermost elastic member of elastic members provide in the lower waist portion. Accordingly, in the lower waist portion, air can easily pass through the space between the skin and the valley, and thus the permeability can be increased. Further, in the lower waist portion, a plurality pleats extending continuously in the up-down direction are easily formed, and thus shock absorbing properties (cushioning properties) in the thickness direction is exhibited in the up-down direction.

### (Example 2)

In an aspect according to Example 2, the outer member includes a front outer member configured to face a front portion of the lower torso of the wearer, and a back outer member configured to face a back portion of the lower torso of the wearer, and a side portion of the front outer member and a side portion of the back outer member are bonded such that the outer member has a tubular shape.

In the aspect according to Example 2, the outer member is divided into a front outer member and a back outer member. Thus, it is not necessary to provide the outer member with a portion facing the crotch. Accordingly, the members of the outer member can be formed in a simpler shape such as a rectangular shape, and thus the outer member can be more easily manufactured.

### (Example 3)

In an aspect according to Example 3, the two sheets include a first sheet and a second sheet, and the first sheet is folded back inward so as to wrap around an upper end of the second sheet, and the elastic members provided in the waist edge portion are arranged between a folded-back portion of the first sheet and the second sheet.

In the aspect according to Example 3, edge elastic members are arranged between a folded-back portion of the first sheet and the second sheet. Conversely, lower elastic members are arranged between a main portion of the first sheet and the second sheet. Therefore, when the outer member is viewed in a cross section, the edge elastic members are disposed inward relative to the lower elastic members and are disposed closer to the skin than the lower elastic members are. Accordingly, the contraction force of the edge elastic members can be exerted more strongly on the skin. Therefore, even if elastic members having a relatively small contraction force are used, the fit of the waist edge portion to the wearer can be improved effectively.

### (Example 4)

In an aspect according to Example 4, the outer member includes a bonded portion where the two sheets are bonded together and/or at least one of the two sheets and the plurality of elastic members are bonded together, and a given imaginary straight line drawn in the up-down direction in the waist edge portion intersects the bonded portion.

In the aspect according to Example 4, when the waist edge portion is viewed in the up-down direction, bonded portions are present at least some positions. Therefore, when the elastic members contract, thereby forming pleats on the sheet, each of the pleats is divided somewhere in the up-down direction. Alternatively, even if each of the pleats is not divided, each of the pleats is bent so as to deviate from the up-down direction. Accordingly, pleats extending continuously in the up-down direction are less likely to be formed in the waist edge portion.

### (Example 5)

In an aspect according to Example 5, an edge elastic member provided in the waist edge portion among the plurality of elastic members is fixed by an adhesive, and a lower elastic member provided in the lower waist portion among the plurality of elastic members is fixed by fusion of the two sheets.

In the aspect according to Example 5, lower elastic members provided in the lower waist portion are fixed by fusing the two sheets instead of using an adhesive. The rigidity of a bonded portion formed by fusing the two sheets is higher than the rigidity of an adjacent non-bonded portion, thereby allowing the sheets to be easily bent at the boundary between the bonded portion and the non-bonded portion. Therefore, pleat tips can easily rise, and thus pleats having tips and valleys extending along the up-down direction can be reliably obtained. As a result, cushioning properties and air permeability can be improved.

Further, the stretchable structure of the outer member provides an excellent fit to the lower torso of the wearer. However, in general, a typical well-fitted stretchable structure tends to require a large force to stretch an outer member in the width direction (in the stretching direction of elastic members) when a diaper is put on or taken off. In particular, if there is a region where elastic members are fixed by an adhesive, stretching of a portion to which the adhesive adheres would be inhibited. In light of this, according to the present aspect, in the lower waist portion, elastic members are fixed by fusing the two sheets instead of using an adhesive, and thus stretching of the elastic members is not inhibited, and the entire outer member can be smoothly stretched even with a small force when the diaper is put on or taken off.

### (Example 6)

In an aspect according to Example 6, the adhesive is applied in a wave shape extending in a stretching direction of the edge elastic member in a plan view, and a waveform of an adhesive provided along one edge elastic member differs from a waveform of an adhesive provided along another edge elastic member that is adjacent to the one edge elastic member in the up-down direction.

In the aspect according to Example 6, adhesives adjacent to each other in the up-down direction have different wave shapes, and thus bonded portions can be formed in any shapes. Accordingly, pleats extending in the up-down direction are less likely to be formed in the waist edge portion, and the above-described effect of preventing excrement from moving in the up-down direction can be improved. Further, the wave shapes of the adhesives extend in the stretching direction of edge elastic members. Therefore, each of pleats formed in the up-down direction is divided at a position in the up-down direction or is bent. Accordingly, movement of excrement along the up-down direction can be prevented and leakage of the excrement can be prevented.

### (Example 7)

In an aspect according to Example 7, a fused portion is formed by the fusion, and the fused portion is formed in a strip shape extending in the up-down direction.

In the aspect according to Example 7, a fused portion extends along the up-down direction. Thus, a long pleat extending along the up-down direction is easily formed, and cushioning properties and the like of the outer member as described above can be more reliably ensured.

This disclosure is based on and claims priority to Japanese Patent Application No. 2021-193831, filed on November 30, 2021.

### List of reference signs

10 outer member
10B back outer member
10F front outer member
11 first sheet
11m first sheet main portion
11e first sheet folded portion
12 second sheet
15 edge elastic member
16 lower elastic member
18 side seal portion
19 waist opening
20 inner member
23 top sheet
24 absorber
24a absorber encapsulating sheet
26 inner gather sheet
27 outer gather sheet
28 liquid impermeable sheet
31 bonded portion
32 non-bonded portion
31A bonded portion by adhesive
31S bonded portion by fusion
40F, 40B adhesion region
60 inner gather
70 outer gather
D1 front-back direction
D2 width direction
A adhesive
B back region
C crotch corresponding region
F front region
U lower waist portion
W waist edge portion
X imaginary line
Z imaginary strip-shaped region

## Claims

1. A pants-type diaper (1) comprising:
an inner member (20) configured to face a crotch of a wearer; and
an outer member (10) configured to be bonded to outer surfaces of a front portion and of a back portion of the inner member (20) and disposed around a lower torso of the wearer,
wherein the outer member (10) includes
a waist edge portion (W) and a lower waist portion (U), the waist edge portion (W) having a waist opening (19) at an upper end thereof and the lower waist portion (U) extending downward from a lower end of the waist edge portion (W),
two sheets and a plurality of elongated elastic members (15, 16), the plurality of elongated elastic members being arranged in a stretched state between the two sheets and being spaced apart from each other in an up-down direction (D4), and
the outer member (10) includes bonded portions (31) where the two sheets are bonded together and/or at least one of the two sheets and the plurality of elongated elastic members (15, 16) are bonded together,
wherein, in a state in which the stretched state is released, pleats having a plurality of tips are formed on an inner side and an outer side of the outer member (10) so as to extend in the up-down direction (D4),
wherein, in the waist edge portion (W), the bonded portions (31) are arranged in a way that a given imaginary straight line (X) drawn in the up-down direction (D4) intersects the bonded portions (31) such that a space corresponding to a valley between adjacent tips of the plurality of tips does not extend continuously from an uppermost elastic member to a lowermost elastic member of the elongated elastic members (15, 16) provided in the waist edge portion (W) among the plurality of elongated elastic members (15, 16), and
wherein, in the lower waist portion (U), the bonded portions (31) extend along the up-down direction (D4) and are spaced apart from each other in a width direction (D2) such that a space corresponding to a valley between adjacent tips of the plurality of tips extends continuously from an uppermost elastic member to a lowermost elastic member of the elongated elastic members (15, 16) provided in the lower waist portion (U) among the plurality of elongated elastic members (15, 16).

2. The pants-type diaper (1) according to claim 1,
wherein
the outer member (10) includes a front outer member (10F) configured to face a front portion of the lower torso of the wearer, and a back outer member (10B) configured to face a back portion of the lower torso of the wearer, and
a side portion of the front outer member (10F) and a side portion of the back outer member (10B) are bonded such that the outer member (10) has a tubular shape.

3. The pants-type diaper (1) according to claim 1 or 2, wherein
the two sheets include a first sheet (11) and a second sheet (12), and the first sheet (11) is folded back inward so as to wrap around an upper end of the second sheet (12), and
the elongated elastic members (15, 16) provided in the waist edge portion (W) are arranged between a folded-back portion of the first sheet (11) and the second sheet (12).

4. The pants-type diaper (1) according to any one of claims 1 to 3, wherein each of the bonded portions (31) is formed in a line shape extending in the up-down direction (D4) as a whole and in the waist edge portion (W), each of the bonded portions (31) is formed in a stepped shape.

5. The pants-type diaper (1) according to any of claims 1 to 4, wherein
an edge elastic member (15) provided in the waist edge portion (W) among the plurality of elongated elastic members (15, 16) is fixed by an adhesive (A), and
a lower elastic member (16) provided in the lower waist portion (U) among the plurality of elongated elastic members (15, 16) is fixed by fusion of the two sheets.

6. The pants-type diaper (1) according to claim 5, wherein
the adhesive (A) is applied in a wave shape extending in a stretching direction of the edge elastic member (15) in a plan view, and
a waveform of an adhesive (A) provided along one edge elastic member (15) differs from a waveform of an adhesive (A) provided along another edge elastic member (15) that is adjacent to the one edge elastic member (15) in the up-down direction (D4).

7. The pants-type diaper (1) according to claim 5 or 6, wherein a fused portion is formed by the fusion, and the fused portion is formed in a strip shape extending in the up-down direction (D4).

## Patentansprüche

1. Windel (1) in Hosenform, umfassend:
ein Innenelement (20), das so ausgebildet ist, dass es dem Hosenschritt eines Trägers zugewandt ist; und
ein Außenelement (10), das so ausgebildet ist, dass es mit den Außenflächen eines vorderen Bereichs und eines hinteren Bereichs des Innenelements (20) verbunden ist und um den Unterkörper des Trägers herum angeordnet ist,
wobei das Außenelement (10) umfasst:
einen Taillenrandbereich (W) und einen unteren Taillenbereich (U), wobei der Taillenrandbereich (W) an seinem oberen Ende eine Taillenöffnung (19) aufweist und sich der untere Taillenbereich (U) von einem unteren Ende des Taillenrandbereichs (W) nach unten erstreckt,
zwei Lagen und eine Vielzahl länglicher elastischer Elemente (15, 16), wobei die Vielzahl länglicher elastischer Elemente in einem gedehnten Zustand zwischen den beiden Lagen angeordnet ist und in einer Auf-Ab-Richtung (D4) voneinander beabstandet ist, und
wobei das Außenelement (10) Verbindungsbereiche (31) aufweist, an denen die beiden Lagen miteinander verbunden sind und/oder mindestens eine der beiden Lagen und die Vielzahl länglicher elastischer Elemente (15, 16) miteinander verbunden sind,
wobei in einem Zustand, in dem der gedehnte Zustand aufgehoben ist, Falten mit einer Vielzahl von Spitzen an einer Innenseite und einer Außenseite des Außenelements (10) so gebildet werden, dass sie sich in der Auf-Ab-Richtung (D4) erstrecken,
wobei im Taillenrandbereich (W) die Verbindungsbereiche (31) so angeordnet sind, dass eine bestimmte imaginäre Gerade (X), die in Auf-Ab-Richtung (D4) gezogen wird, die Verbindungsbereiche (31) derart schneidet, dass sich ein Raum, der einer Vertiefung zwischen benachbarten Spitzen der Vielzahl von Spitzen entspricht, nicht durchgehend von einem obersten elastischen Element bis zu einem untersten elastischen Element der Vielzahl länglicher elastischer Elemente (15, 16) erstreckt, die im Taillenrandbereich (W) unter der Vielzahl länglicher elastischer Elemente (15, 16) angeordnet sind, und
wobei sich im unteren Taillenbereich (U) die Verbindungsbereiche (31) entlang der Auf-Ab-Richtung (D4) erstrecken und in einer Breitenrichtung (D2) so voneinander beabstandet sind, dass sich ein Raum, der einer Vertiefung zwischen benachbarten Spitzen der Vielzahl von Spitzen entspricht, durchgehend von einem obersten elastischen Element bis zu einem untersten elastischen Element der länglichen elastischen Elemente (15, 16), die im unteren Taillenbereich (U) unter der Vielzahl länglicher elastischer Elemente (15, 16) angeordnet sind, durchgehend erstreckt.

2. Windel (1) in Hosenform nach Anspruch 1, wobei
das Außenelement (10) ein vorderes Außenelement (10F), das so ausgebildet ist, dass es einem vorderen Bereich des Unterkörpers des Trägers zugewandt ist, und ein hinteres Außenelement (10B) umfasst, das so ausgebildet ist, dass es einem hinteren Bereich des Unterkörpers des Trägers zugewandt ist, und
ein Seitenbereich des vorderen Außenelements (10F) und ein Seitenbereich des hinteren Außenelements (10B) so miteinander verbunden sind, dass das Außenelement (10) eine röhrenförmige Gestalt aufweist.

3. Windel (1) in Hosenform nach Anspruch 1 oder 2, wobei
die beiden Lagen eine erste Lage (11) und eine zweite Lage (12) umfassen, und die erste Lage (11) nach innen zurückgefaltet ist, um ein oberes Ende der zweiten Lage (12) zu umschließen, und
die im Taillenrandbereich (W) angeordneten länglichen elastischen Elemente (15, 16) zwischen einem zurückgefalteten Bereich der ersten Lage (11) und der zweiten Lage (12) angeordnet sind.

4. Windel (1) in Hosenform nach einem der Ansprüche 1 bis 3, wobei jeder der Verbindungsbereiche (31) in einer sich insgesamt in der Auf-Ab-Richtung (D4) erstreckenden Linienform ausgebildet ist und im Taillenrandbereich (W) jeder der Verbindungsbereiche (31) stufenförmig ausgebildet ist.

5. Windel (1) in Hosenform gemäß einem der Ansprüche 1 bis 4, wobei
ein im Taillenrandbereich (W) angeordnetes elastisches Randelement (15) aus der Vielzahl länglicher elastischer Elemente (15, 16) durch einen Klebstoff (A) befestigt ist, und
ein unteres elastisches Element (16), das im unteren Taillenbereich (U) unter der Vielzahl länglicher elastischer Elemente (15, 16) angeordnet ist, durch Verschmelzen der beiden Lagen befestigt ist.

6. Windel (1) in Hosenform nach Anspruch 5, wobei
der Klebstoff (A) in einer Wellenform aufgetragen ist, die sich in der Draufsicht in einer Dehnungsrichtung des elastischen Randelements (15) erstreckt, und
sich die Wellenform eines entlang eines elastischen Randelements (15) aufgebrachten Klebstoffs (A) von der Wellenform eines entlang eines anderen elastischen Randelements (15) aufgebrachten Klebstoffs (A) unterscheidet, das in der Auf-Ab-Richtung (D4) an das eine elastische Randelement (15) angrenzt.

7. Windel (1) in Hosenform nach Anspruch 5 oder 6, wobei durch das Verschmelzen ein verschmolzener Bereich gebildet ist und der verschmolzene Bereich in Form eines Streifens ausgebildet ist, der sich in der Auf-Ab-Richtung (D4) erstreckt.

## Revendications

1. Couche-culotte (1), comprenant :
une partie interne (20), conçue pour être tournée vers la région de l'entrejambe d'un porteur ; et
une partie externe (10), conçue pour être reliée aux surfaces externes d'une section avant et d'une section arrière de la partie interne (20) et pour entourer le tronc inférieur du porteur,
dans laquelle la partie externe (10) comprend :
une section de bord de taille (W) et une section inférieure de taille (U), la section de bord de taille (W) présentant, à son extrémité supérieure, une ouverture de taille (19), et la section inférieure de taille (U) s'étendant vers le bas depuis une extrémité inférieure de la section de bord de taille (W), et
deux nappes ainsi qu'une pluralité d'éléments élastiques allongés (15, 16), la pluralité d'éléments élastiques allongés étant disposée, à l'état étiré, entre les deux nappes, et étant espacée dans une direction haut-bas (D4),
la partie externe (10) comprend sections de liaison (31), au niveau desquelles les deux nappes sont reliées l'une à l'autre et/ou au niveau desquelles au moins l'une des deux nappes est reliée à la pluralité d'éléments élastiques allongés (15, 16),
dans laquelle, dans un état où l'état étiré est supprimé, des plis présentant une pluralité de sommets sont formés sur une face interne et une face externe de la partie externe (10), lesdits plis s'étendant dans la direction haut-bas (D4),
dans laquelle, dans la section de bord de taille (W), les sections de liaison (31) sont disposées de telle sorte qu'une ligne droite imaginaire prédéterminée (X), qui s'étend dans la direction haut-bas (D4), coupe les sections de liaison (31) de telle sorte qu'un espace intermédiaire correspondant à une vallée entre des sommets adjacents de la pluralité de sommets ne s'étend pas de manière continue depuis l'élément élastique le plus supérieur jusqu'à l'élément élastique le plus inférieur des éléments élastiques allongés (15, 16) prévus dans la section de bord de taille (W) parmi la pluralité d'éléments élastiques allongés (15, 16), et
dans laquelle, dans la section inférieure de taille (U), les sections de liaison (31) s'étendent le long de la direction haut-bas (D4) et sont espacées les unes des autres dans une direction de largeur (D2), de sorte qu'un espace intermédiaire correspondant à une vallée entre des sommets adjacents de la pluralité de sommets s'étend de manière continue depuis l'élément élastique le plus supérieur jusqu'à l'élément élastique le plus inférieur des éléments élastiques allongés (15, 16) prévus dans la section inférieure de taille (U) parmi la pluralité d'éléments élastiques allongés (15, 16).

2. La couche-culotte (1) selon la revendication 1, dans laquelle
la partie externe (10) comprend une partie externe avant (10F), conçue pour être tournée vers une section avant du tronc inférieur du porteur, ainsi qu'une partie externe arrière (10B), conçue pour être tournée vers une section arrière du tronc inférieur du porteur, et
une section latérale de la partie externe avant (10F) est reliée à une section latérale de la partie externe arrière (10B) de telle sorte que la partie externe (10) présente une forme tubulaire.

3. La couche-culotte (1) selon la revendication 1 ou 2, dans laquelle
les deux nappes comprennent une première nappe (11) et une deuxième nappe (12), et la première nappe (11) est repliée vers l'intérieur de manière à entourer une extrémité supérieure de la deuxième nappe (12), et
les éléments élastiques allongés (15, 16) prévus dans la section de bord de taille (W) sont disposés entre une section repliée de la première nappe (11) et de la deuxième nappe (12).

4. La couche-culotte (1) selon l'une quelconque des revendications 1 à 3, dans laquelle chacune des sections de liaison (31) est formée, dans son ensemble, sous la forme d'une ligne s'étendant dans la direction haut-bas (D4), et dans laquelle, dans la section de bord de taille (W), chacune des sections de liaison (31) est formée en forme d'escalier.

5. La couche-culotte (1) selon l'une quelconque des revendications 1 à 4, dans laquelle
un élément élastique de bord (15), prévu dans la section de bord de taille (W) parmi la pluralité d'éléments élastiques allongés (15, 16), est fixé au moyen d'un adhésif (A), et
un élément élastique inférieur (16), prévu dans la section inférieure de taille (U) parmi la pluralité d'éléments élastiques allongés (15, 16), est fixé par fusion des deux nappes.

6. La couche-culotte (1) selon la revendication 5, dans laquelle
l'adhésif (A) est appliqué, en vue en plan, selon une forme ondulée le long d'une direction d'étendue de l'élément élastique de bord (15), et
la forme ondulée d'un adhésif (A) prévu le long d'un élément élastique de bord (15) est différente de la forme ondulée d'un adhésif (A) prévu le long d'un autre élément élastique de bord (15), adjacent audit élément élastique de bord (15) dans la direction haut-bas (D4).

7. La couche-culotte (1) selon la revendication 5 ou 6, dans laquelle une section de fusion est formée par ladite fusion, et la section de fusion est formée sous une forme en bande s'étendant dans la direction haut-bas (D4).
